(19) Europäisches Patentamt
European Patent Office
Office européen des brevets

(11) Veröffentlichungsnummer: **0 360 130**
**A2**

# (12) EUROPÄISCHE PATENTANMELDUNG

(21) Anmeldenummer: **89116831.2**

(22) Anmeldetag: **12.09.89**

(51) Int. Cl.5: **C07H 15/14 , C07D 321/00 , C07H 17/04 , A61K 31/70 , A61K 31/335**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4137 und DSM 3816.

(30) Priorität: **17.09.88 DE 3831695**

(43) Veröffentlichungstag der Anmeldung: **28.03.90 Patentblatt 90/13**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Hammann, Peter, Dr.**
**Mörikestrasse 6**
**D-6233 Kelkheim(Taunus)(DE)**
Erfinder: **Kretzschmar, Gerhard, Dr.**
**Ulmenweg 10**
**D-6236 Eschborn 2(DE)**

(54) **Elaiophylinderivate, Verfahren zu ihrer Herstellung, ihre Verwendung als Arzneimittel und sie enthaltende Arzneimittel.**

(57) Die Erfindung betrifft Elaiophylinderivate der Formel I, II und III

EP 0 360 130 A2

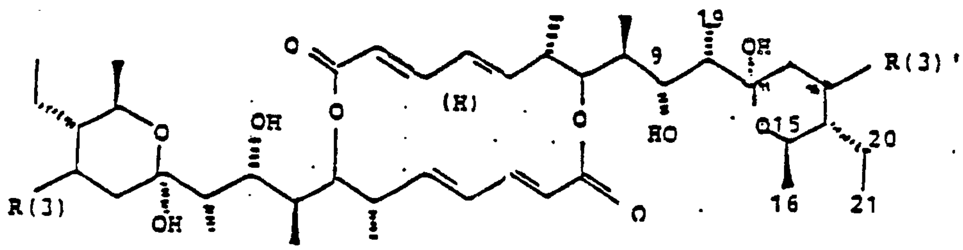

(I)

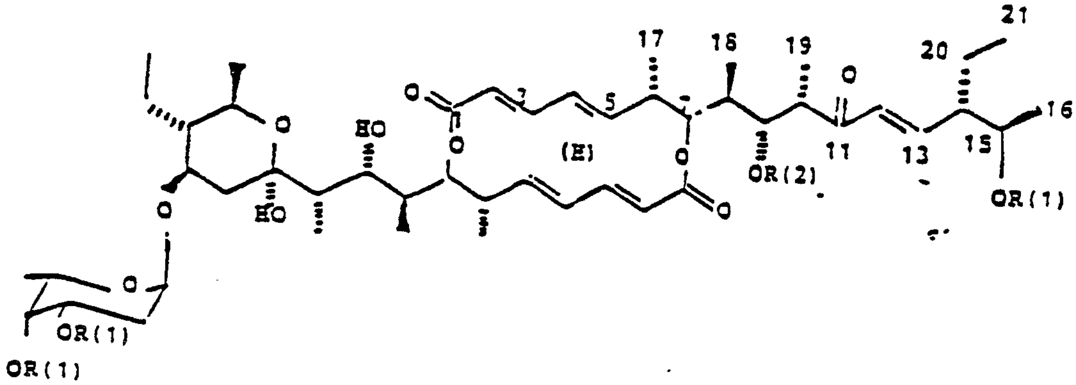

II

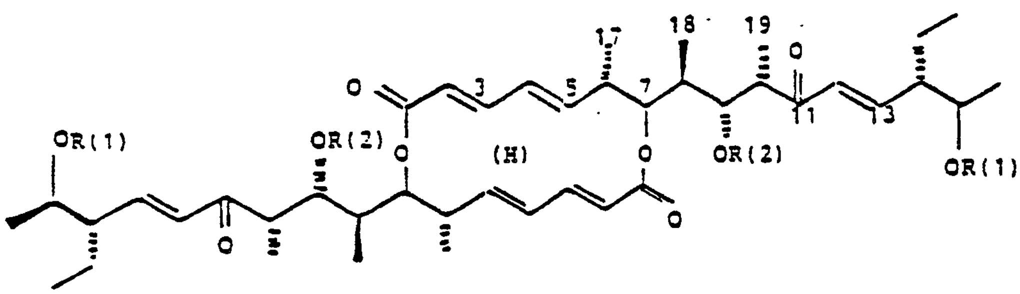

III

in denen die Substituenten R(1), R(2), R(3) und R(3′) die angegebenen Bedeutungen haben. Die Verbindungen zeigen antibakterielle und antivirale Aktivität.

# Elaiophylinderivate, Verfahren zu ihrer Herstellung ihre Verwendung als Arzneimittel und sie enthaltende Arzneimittel

Die Erfindung betrifft Elaiophylinderivate sowie ihre Herstellung und ihre Verwendung als Arzneimittel insbesondere als antibakteriell und antiviral wirksame Arzneimittel. Eine Abspaltung der 2,6-Desoxyfucose von Elaiophylin bereitet aufgrund der Instabilität des Moleküls sowohl unter sauren als auch unter basischen Bedingungen Schwierigkeiten, siehe dazu D. Seebach, Liebigs Ann. Chem. 1986, S. 1281 und W. Keller-Schierlein, Helv. Chim. Acta 64, 407 (1981), S. Takahashi Chem. Pharm. Bull. 15, 1651, 1657, 1726 (1976). Die Erfindung stellt nun erstmals Bedingungen zur Verfügung, unter denen aus Elaiophylin

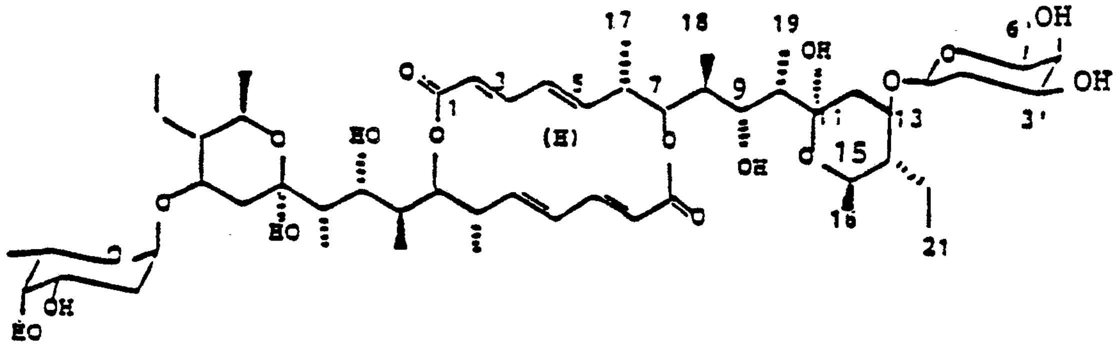

durch kontrollierte basische Reaktionsbedingungen eine Abspaltung der Desoxyfucose zu den unsymmetrischen Enonen II

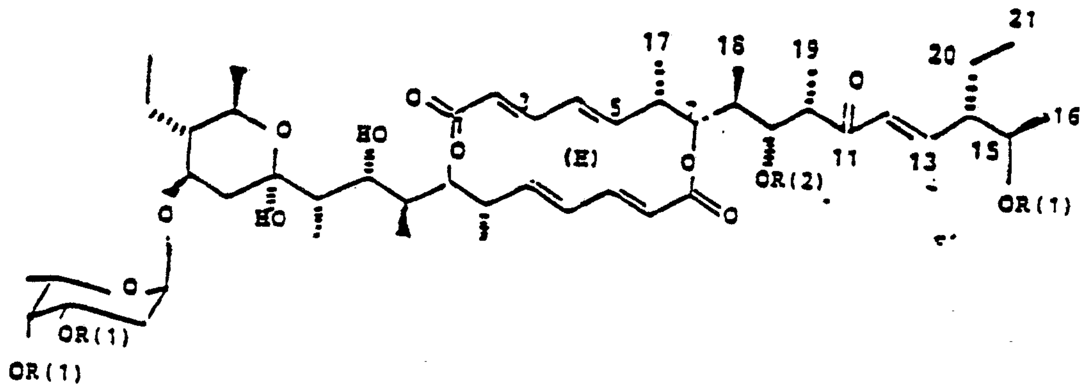

II

und den symmetrischen Enonen III

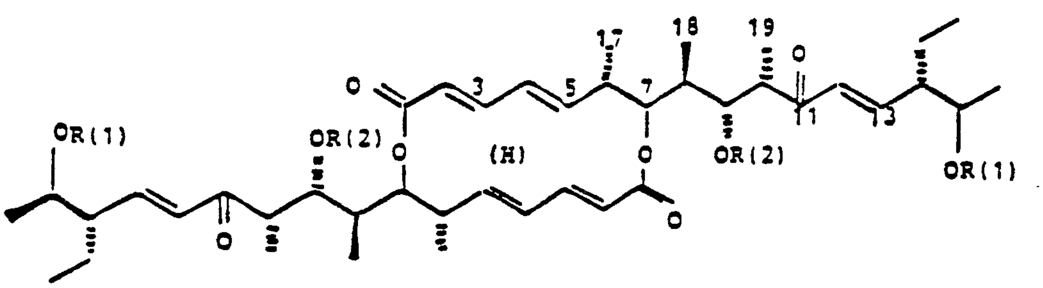

III

in hohen Ausbeuten gelingt.

Durch Addition von Thioverbindungen an III entstehen Elaiophylinderivate I

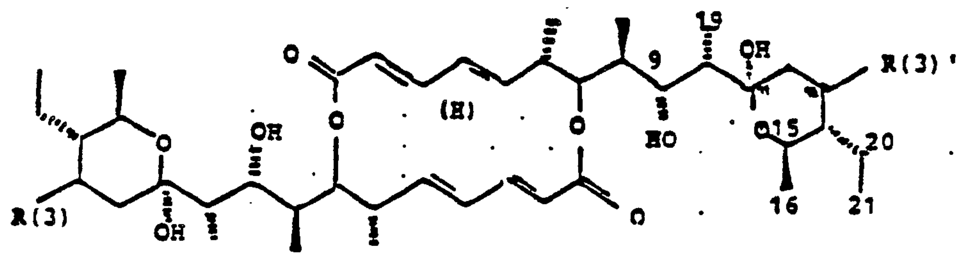

I

bzw. durch Hydrierung entstehen die entsprechenden Octahydro-Verbindungen I mit R(3) = H und R(3′) = H bzw. R(3) = L-Desoxyfucose und R(3′) = H.

Die Erfindung betrifft somit Verbindungen I

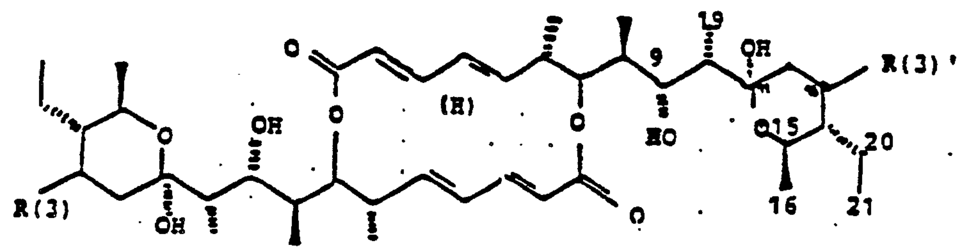

I

wobei die C-C-Doppelbindungen in dem Macrodiolidring der Verbindung der Formel I auch hydriert sein können, in welcher Formel I bedeuten:

a) R(3) und R(3′), gleich oder verschieden, einen Rest der Formel IV -SR(4) (IV)

mit R(4) gleich Wasserstoff, ($C_1$-$C_{10}$)-Alkyl, das unsubstituiert oder mit OH oder COOH substituiert ist, ($C_2$-$C_{10}$)-Alkenyl, ($C_3$-$C_8$)-Cycloalkyl, Pyrrolyl, Benzpyrrolyl, Imidazolyl, Benzimidazolyl, Triazolyl, Tetrazolyl, Phenyl, wobei die aromatischen oder Heteroarylreste unsubstituiert sind oder durch ($C_1$-$C_4$)-Alkyl, ($C_1$-$C_4$)-Alkoxy, ($C_1$-$C_4$)-Alkylcarbonyl, Carboxyl, F, Cl, Br, I, $NO_2$ oder CN ein- oder zweifach substituiert sind.

b) R(3) L-Desoxyfucose und R(3)′ wie unter a) definiert.

c) wenn die C-C-Doppelbindungen im Makrodiolidring hydriert sind:

R(3) und R(3)′ Wasserstoff oder

R(3) L-Desoxyfucose und R(3)′ Wasserstoff

Bevorzugt sind Verbindungen I in denen R(3) und R(3)′ einen Rest der Formel IV darstellen mit R(4) gleich Wasserstoff,

($C_1$-$C_5$)-Alkyl, das unsubstituiert oder mit OH oder COOH substituiert ist,

($C_2$-$C_5$)-Alkenyl, ($C_5$-$C_8$)-Cycloalkyl, Pyrrolyl, Benzpyrrolyl, Imidazolyl, Phenyl, das unsubstituiert ist oder substituiert durch ($C_1$-$C_4$)-Alkyl, ($C_1$-$C_4$)-Alkoxy, F, Cl, Br, $NO_2$ oder CN.

Insbesondere sind solche Verbindungen I bevorzugt, in denen R(3) die L-Desoxyfucosylgruppe und R-(3)′ einen Rest SR(4) IV,

wie oben definiert, ist.

Ganz besonders bevorzugt sind Verbindungen I in denen der Makrodiolidring hydriert ist und in denen R(3) der L-Desoxyfucosylrest oder Wasserstoff und R(3)′ Wasserstoff ist.

Die Erfindung betrifft weiterhin ein Verfahren zum Herstellen einer Verbindung I,nach welchem man

a) Elaiophylin

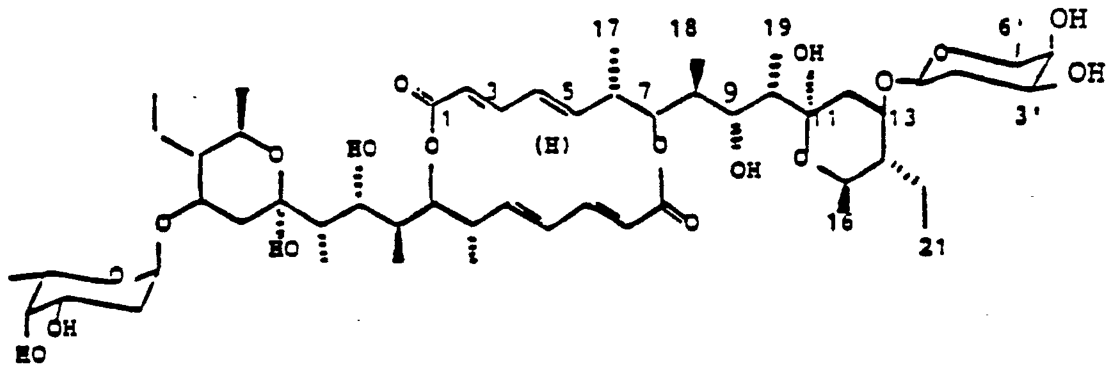

mit einer Base in wässrigem Lösungsmittel bei einem pH-Wert von 8 bis 11 zu einer Verbindung II

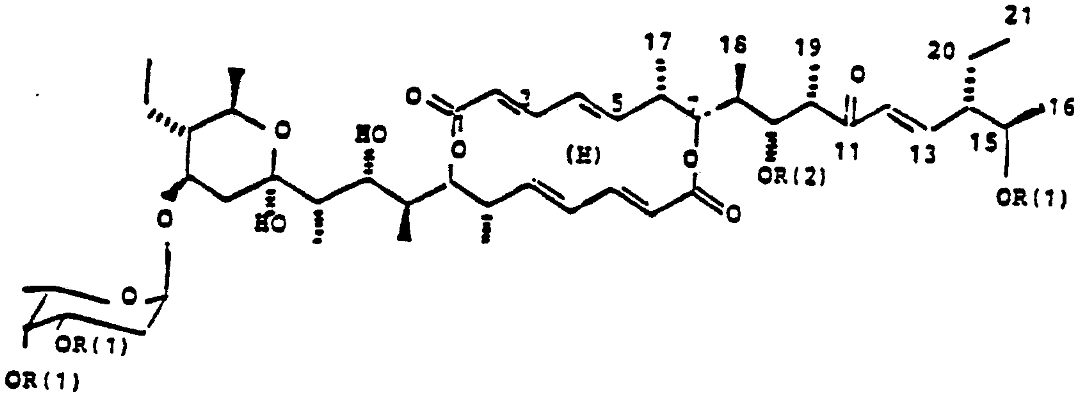

II

oder III

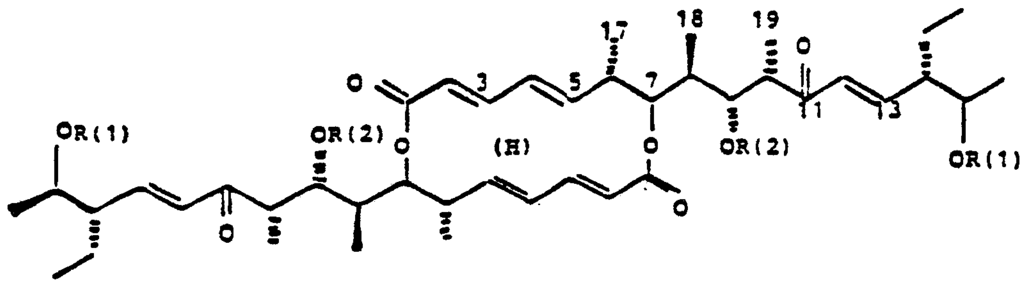

III

umsetzt
und die so erhaltene Verbindung II oder III in der R(1) = R(2) = H ist, mit einem Thiol entweder der Formel IV
HSR(4) IV,
in der R(4) wie in Anspruch 1 definiert ist, zu einer Verbindung I umsetzt
oder
die so erhaltene Verbindung II oder III mit R(1) = R(2) = H vollständig hydriert, wobei man aus Verbindungen II Verbindungen I mit R(3) = L-Desoxyfucose und R(3)' gleich Wasserstoff und aus Verbindungen III mit R(1) = R(2) = Wasserstoff Verbindungen I mit R(3) = R(3)' = Wasserstoff erhält;
oder daß man
b) eine Verbindung II

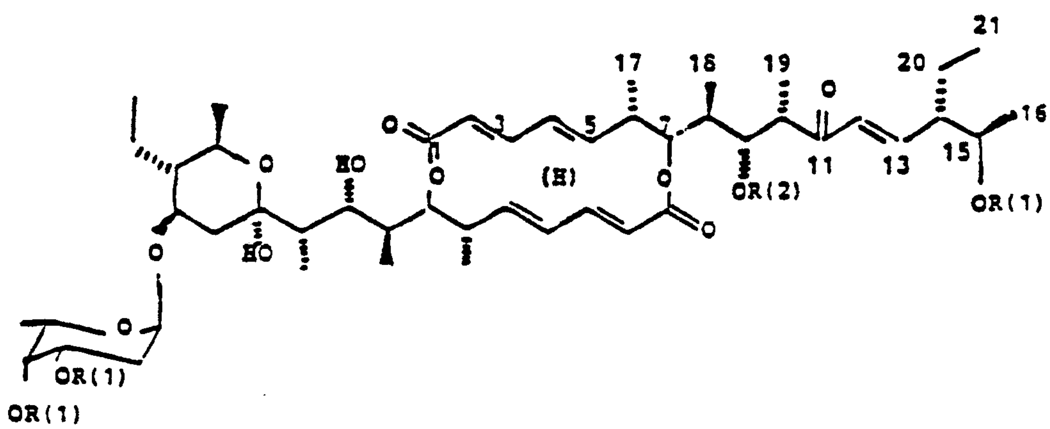

II

oder III

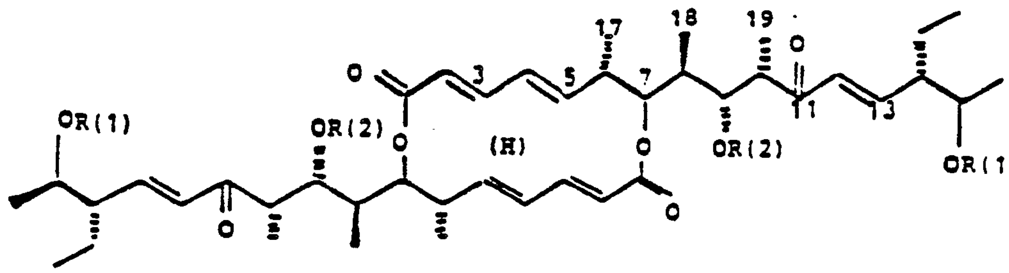

III,

in der R(1) = R(2) = H ist, mit einem Thiol der Formel
HSR(4)
umsetzt, in welchem R(4) wie in Anspruch 1 definiert ist, wobei man aus Verbindung II die Verbindung I mit R(3) = L-Desoxyfucose und R(3)′ = SR(4) IV erhält; und aus Verbindung III die Verbindung I mit R(3) = R(3)′ = SR(4); oder daß man

c) eine Verbindung II oder III, in der die C-C-Doppelbindungen in dem Makrodiolidring auch hydriert sein können, mit R(1) = R(2) = H vollständig hydriert, wobei man aus Verbindungen II Verbindungen I mit R(3) = L-Desoxyfucose und R(3) gleich Wasserstoff und aus Verbindungen III mit R(1) = R(2) = Wasserstoff Verbindungen I mit R(3) = R(3)′ = Wasserstoff erhält.

Die Erfindung betrifft desweiteren Verbindungen III

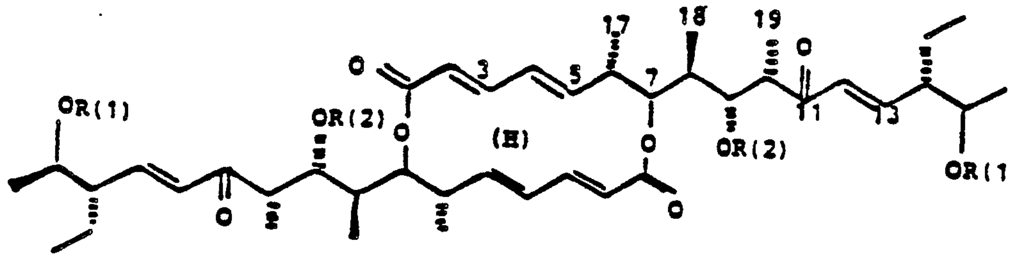

III,

in welchen die folgenden Substituenten folgende Bedeutung haben R(1) und R(2), gleich oder verschieden, Wasserstoff, einen Rest der Formel V oder V′

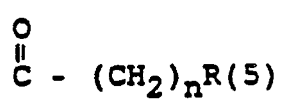

V

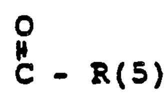

V'

mit n = 1-3 und
R(5) gleich ($C_1$-$C_5$)-Alkyl, ($C_2$-$C_{15}$)-Alkenyl, ($C_2$-$C_{15}$)-Alkinyl, ($C_3$-$C_9$)-Cycloalkyl, Phenyl, Naphthyl, Furyl, Thienyl, unsubstituiert oder durch F, Cl, Br, J, $NO_2$, CN, OH, COOH, ($C_1$-$C_4$)-Alkyl oder ($C_1$-$C_4$-Alkoxy substituiert, und wobei für den Fall R(1) gleich H auch R(2) gleich H ist,
einen Rest der Formel VI
$SO_2R(6)$ VI
mit R(6) gleich ($C_1$-$C_{10}$)-Alkyl, Phenyl, Tolyl, wobei für den Fall R(1) gleich H auch R(2) gleich H ist.

Außerdem ist die Erfindung auf Verbindungen II gerichtet

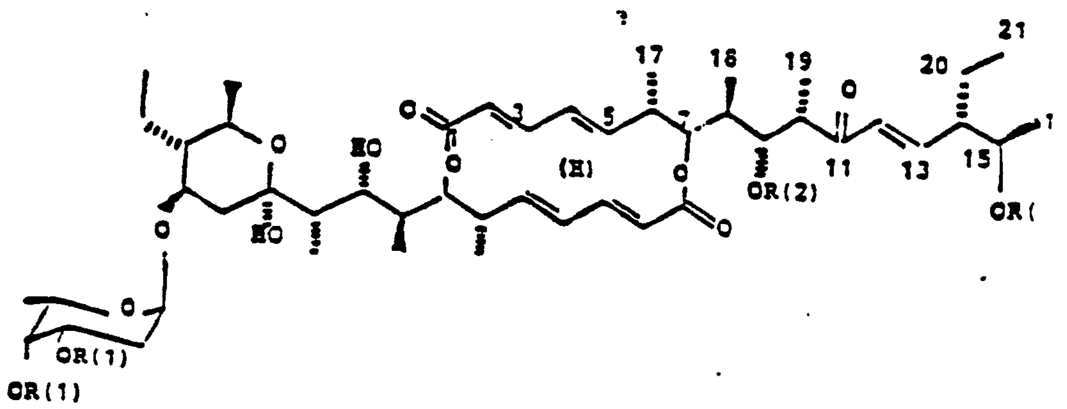

II

in welchen folgende Substituenten folgende Bedeutung haben
R(1) und R(2), gleich oder verschieden, Wasserstoff, einem Rest der Formel V oder V′

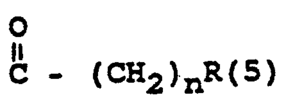

V

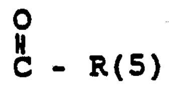

V'

mit n = 1-3 und
R(5) gleich ($C_1$-$C_5$)-Alkyl, ($C_2$-$C_{15}$)-Alkenyl, ($C_2$-$C_{15}$)-Alkinyl, ($C_3$-$C_9$)-Cycloalkyl, Aryl, Heteroaryl, unsubstituiert oder durch Halogen, $NO_2$, CN, OH, COOH, ($C_1$-$C_4$)-Alkyl oder ($C_1$-$C_4$-Alkoxy substituiert, und wobei für den Fall R(1) gleich H auch R(2) gleich H ist,
einen Rest der Formel VI
$SO_2R(6)$   VI
mit R(6) gleich ($C_1$-$C_{10}$)-Alkyl, Phenyl, Tolyl, wobei für den Fall R(1) gleich H auch R(2) gleich H ist.

Bevorzugt sind auch Zwischenverbindungen II und III in denen R(1) und R(2) = Wasserstoff sind oder unabhängig voneinander einen Rest der Formel V darstellen, in denen R(5) die Bedeutung ($C_1$-$C_5$)-Alkyl, ($C_5$-$C_6$)-Cycloalkyl oder Phenyl hat, wobei für den Fall R(1) = Wasserstoff R(2) auch Wasserstoff ist. Ebenfalls bevorzugt sind Verbindungen II und III, in denen R(1) einen Rest der Formel VI darstellt mit R(6) = ($C_1$-$C_5$)-Alkyl,Phenyl oder Tolyl.
Alle diese Verbindungen besitzen eine antibakterielle Aktivität gegen humanpathogene Bakterien sowie antivirale Aktivität.

Die Verbindungen I, II und III werden nach verschiedenen Verfahren erhalten.

a. Elaiophylin wird in einem Lösungsmittelgemisch mit einer Base umgesetzt, die einen pH-Wert von 8 bis 11 aufweist, wobei durch $\beta$-Eliminierung die L-Desoxyfucose zu Verbindung II (R(1), R(2) = H) und Verbindung III (R(1), R(2) = H) eliminiert wird.

b. Wird Verbindung II oder III mit R(1) = R(2) = Wasserstoff mit einem Thiol der Formel HSR(4), wie oben definiert umgesetzt, so erhält man durch nukleophile Addition an das Enonsystem die entsprechenden Verbindungen I. So entsteht aus II die Verbindung I mit R(3) = L-Desoxyfucose and R(3)′ = IV und aus III entsteht die Verbindung I mit R(3) = R(3)′ = IV.

c. Wird Verbindung II oder III mit R(1) = R(2) = H mit Wasserstoff vollständig hydriert, so erhält man aus II die Verbindung I mit R(3) = L-Desoxyfucose und R(3)′ = Wasserstoff und aus Verbindung III erhält man Verbindung I mit R(3) = R(3)′ = Wasserstoff (Octahydrodidesoxyelaiophyliden).

d. Wird Verbindung II oder III mit R(1) = R(2) = Wasserstoff oder R(1) = V bzw. V′, wie oben definiert, oder R(1) = VI, wie oben definiert, und R(2) = Wasserstoff mit einer Verbindung der Formel VII oder VII′

$$Q-\overset{O}{\overset{\|}{C}}-R(5) \qquad\qquad Q-\overset{O}{\overset{\|}{C}}-(CH_2)_n-R(5)$$

VII VII'

in denen n und R(5) die unter V und V′ angegebene Bedeutung hat, und Q = Chlorid, Bromid, Imidazolid oder Säureanhydrid bedeutet, umgesetzt, so erhält man aus Verbindungen II oder III mit R(1) = R(2) = H eine Verbindung mit R(1) = V oder V′ und R(2) = Wasserstoff bzw. Verbindungen mit R(1), R(2) = V bzw. V′ und aus Verbindungen II oder III mit R(1) = V und R(2) = Wasserstoff Verbindungen mit R(1) = V oder V′ und R(2) = V oder V′, in denen R(1) und R(2) gleich oder verschieden sind.

e. Wird eine Verbindung II oder III mit R(1) = R(2) = Wasserstoff oder R(1) = V bzw. V′, wie oben definiert, oder R(1) = VI, wie oben definiert, und R(2) = Wasserstoff mit einer Verbindung der Formel $ClSO_2R(6)$ umgesetzt, so erhält man aus Verbindung II oder III mit R(1) = R(2) = Wasserstoff Verbindungen mit R(1) = VI und R(2) = Wasserstoff oder R(1) = R(2) = VI, bzw. aus Verbindungen II oder III mit R-(1) = V bzw. V′, R(2) = Wasserstoff die Derivate mit R(1) = V bzw. V′ und R(2) = VI sowie aus R(1) = VI und R(2) = Wasserstoff die Derivate mit R(1) = VI und R(2), wobei R(1) und R(2) gleich oder verschieden sein können.

Im folgenden werden die Verfahren a bis e näher beschrieben. Mit Hilfe der Variante a kann aus Elaiophylin die L-Desoxyfucose-Seitenkette eliminiert werden. Es entstehen die Verbindungen II und III mit R(1) = R(2) = Wasserstoff. Bei höheren Temperaturen und längeren Reaktionszeiten nimmt die Ausbeute an II ab und die an III zu.

Man geht am besten so vor, daß man Elaiophylin in einem Gemisch aus Wasser, Alkohol und einem inerten organischen Lösungsmittels suspendiert. Als Alkohole eignen sich Methanol, Ethanol und Isopropanol, als inerte Lösungsmittel Chloroform, Essigester, THF, Methylenchlorid.

Bevorzugt ist die Mischung aus Wasser, Ethanol und Essigester. Die Reaktionstemperaturen liegen zwischen 20 °C und dem Siedepunkt der Reaktionsmischung. Bevorzugt wird die Reaktion unter Rückfluß durchgeführt. Die Reaktionszeiten betragen 0,5 bis 180 Stunden, bevorzugt 1 bis 24 Stunden. Die Beendigung der Reaktion wird mittels Dünnschichtchromatographie bestimmt.

Als Basen finden Alkali- und Erdalkalihydroxide und -carbonate und -hydrogencarbonate Verwendung. Der pH-Wert der Reaktionslösung sollte zwischen 7 und 14 liegen, vorzugsweise zwischen 8 und 11. Ganz besonders bevorzugt sind Natriumhydrogencarbonat und Kaliumhydrogencarbonat bei einm pH-Wert von 8 bis 11. Eine Regulierung des pH-Wertes kann mit HCl oder $H_2SO_4$ erfolgen oder über einen entsprechenden Puffer.

Das als Ausgangssubstanz benötigte Elaiophylin kann nach bekannten Herstellungsverfahren hergestellt werden. Z. B. kommt das nach der deutschen Patentanmeldung P 3721722.4 in Frage. Dabei fällt Elaiophylin als Fermentationsprodukt von Kulturen der Stämme DSM 4137 und DSM 3816 an.

Beim Verfahren b wird Verbindung II oder III mit R(1) = R(2) = Wasserstoff in einem Lösungsmittel, wie Alkohol oder $CHCl_3$, $CH_2Cl_2$, THF, Dioxan, gelöst und mit äquimolaren Mengen oder mit einem bis zu 50fachen Überschuß an Thiol der Formel $HSR_4$ in Gegenwart einer Base umgesetzt.
Als Basen eignen sich vor allem organische Amine, bevorzugt Triethylamin, als Akohole finden vor allem Methanol, Ethanol oder Isopropanol Verwendung.
Werden direkt Salze $M^+ {}^-SR(4)$ eingesetzt, mit $M^+$ z. B. gleich Natrium$^+$ oder Calium$^+$, so eignet sich THF oder Dioxan als Lösungsmittel. Diese Salze werden dann in THF oder Dioxan aus HSR(4) durch Zugabe der entsprechenden Metallhydride hergestellt. Die Reaktionstemperaturen liegen zwischen 0 °C und dem Siedepunkt des Lösungsmittels. Bevorzugt sind Temperaturen zwischen 50 °C und dem Siedepunkt. Die Reaktionszeiten betragen 0,1 -180 Stunden, bevorzugt 0,1 bis 120 Stunden in Abhängigkeit von R(4).

Mit Hilfe der Verfahrenvarianten c) lassen sich die C-C-Doppelbindungen des Macrodiolidrings sowie die Enon-Doppelbindung in II und III ($R_1 = R_2 = H$) hydrieren.

Bei der Verfahrensvarianten c) geht man am besten so vor, daß man das zu hydrierende Elaiophylinderivat II $R_1/R_2 = H$ oder III $R_1/R_2 = H$, bevorzugt gelöst in einem Lösungsmittel wie Methanol, Ethanol, Isopropanol oder Ethylacetat oder einem Gemisch dieser Lösungsmittel oder einem wäßrigen Gemisch dieser Lösungsmittel, in Gegenwart eines gängigen Hydrierkatalysators nach literaturbekannten Hydrierkatalysatoren mit Wasserstoff umsetzt. Gängige Hydrierkatalysatoren sind beispeislweise Elemente der 8. Gruppe wie Platin, Palladium oder auch Nickel, die meist zum Zwecke der Vergrößerung der reaktiven Oberfläche, beispielsweise auf Aktivkohle-, Siliziumdioxid-oder Aluminiumoxidträgern aufgetragen sind. Wird die Reaktion in einem absoluten primären Alkohol als Lösungsmittel durchgeführt, so erhält man außer

einer Hydrierung der C=C-Doppelbindungen auch eine Ketalisierung zu den $C_{11}/C_{11}'$-Di-O-Alkylen.

Je nach verwendetem Katalysator kann die Reaktion sowohl ohne als auch mit Überdruck an Wasserstoff, beispielsweise bis zu 1 Atmosphäre, durchgeführt werden. Die Reaktionstemperaturen liegen zwischen 0°C und 40°C, vorzugsweise bei Zimmertemperatur. Die Reaktionszeiten sind abhängig von der Ansatzgröße und der Konzentration der zu reduzierenden Verbindung. Solche Hydrierungsverfahren sind beispielsweise beschrieben in Organikum, Organisch Chemisches Grundpraktikum, 15. Auflage, VEB Deutscher Verlag der Wissenschaften, Berlin, 1976, S. 359-371.

Mit Hilfe der Verfahrensvariante d) können die Hydroxylgruppen in Formel II und III verestert werden. Da die Reaktionsgeschwindigkeit der Veresterung an 3′, 4′ und 15 Position - also für die $R^1$-Derivate - größer ist, als die Veresterung an 9-Position, eröffnet sich die Möglichkeit für $R^1$ und $R^2$ sowohl identische, also auch unterschiedliche Substituenten vorzusehen.

Bei höheren Temperaturen und/oder genügend langen Reaktionszeiten wird auch die OH-Gruppe in 9-Position verestert. Dies bedeutet aber auch, daß eine Veresterung in 9-Position nur nach vorheriger Veresterung der OH-Gruppen in 3′, 4′ und 15-Position möglich ist. Auf diese Weise können also beispielsweise zunächst die OH-Gruppen in 3′, 4′ und 15-Position verestert werden und anschließend, gegebenenfalls nach Isolierung und Reinigung des Produktes, kann dann in einer zweiten Reaktion entsprechend Verfahrensvariante a die OH-Gruppe in 9-Stellung verestert werden.

Bei der Verfahrensvariante b) geht man am besten so vor, daß eine Verbindung der Formel II oder III, worin $R^1$, $R^2$ die oben angegebenen Bedeutungen haben, in äquimolaren Mengen oder in bis zu einem 50-fachen Überschuß, gegebenenfalls in einem inerten aprotischen Lösungsmittel wie Chloroform, Methylenchlorid, Tetrahydrofuran (THF), Essigester oder Dioxan, mit einer Verbindung der Formel VII oder VII′ bis zur Beendigung der Reaktion umsetzt, gegebenenfalls in Gegenwart einer Base, vorzugsweise Pyridin.

Die Reaktionstemperaturen liegen dabei zwischen -70°C und +100°C, vorzugsweise bei Verwendung eines Lösungsmittels zwischen dem Festpunkt und dem Siedepunkt des Lösungsmittels, insbesondere zwischen -70°C und +40°C. Die Reaktionszeiten betragen 1 bis 180 Stunden, bevorzugt 1 bis 48 Stunden, besonders bevorzugt 1 bis 8 Stunden. Die Beedigung der Reaktion kann beispielsweise mittels Dünnschichtchromatographie bestimmt werden (DC-Kontrolle).

Die Ausgangsverbindungen für die Verfahrensvariante d), die Verbindungen der Formel VII und/oder VII′ sind, sofern nicht käuflich, auf einfache Weise nach literaturbekannten Verfahren herstellbar. Beispielsweise erhält man die Säurechloride durch Umsetzung der entsprechenden Carbonsäure mit Thionylchlorid, $PCl_3$ oder $PCl_5$. Solche Verfahren sind beispielsweise beschrieben in Gattermann/Wieland, "Die Praxis des Organischen Chemikers", 43. Auflage, Walter de Gruyter, Berlin, New York 1982, S. 303 ff.

Bei der Verfahrensvarianten e) geht man am besten vor, daß man das ein Elaiophylin-Derivat in II oder III äquimolaren Mengen oder in einem bis zu 50-fachen Überschuß umsetzt mit Sulfonsäurehalogeniden der Formel $Y\text{-}SO_2R^6$.

Gegebenenfalls kann auch diese Umsetzung unter Basenzusatz erfolgen. Als Basen kommen beispielsweise Triethylamin, Pyridin oder Lutidin in Frage. Eine Variante des Verfahrens e) besteht darin, daß man in einem geeigneten, vorzugsweise inerten Lösungsmittel wie Chloroform, Methylenchlorid, THF, Essigester oder Dioxan arbeitet. Auch hier kann der Überschuß der oben aufgeführten Verbindungen bis zur 50-fachen Menge betragen.

Die Reaktionstemperaturen liegen dabei zwischen -70°C und +100°C, vorzugsweise bei Verwendung eines Lösungsmittels zwischen dem Festpunkt und dem Siedepunkt es Lösungsmittels, insbesondere zwischen -70°C und -40°C. Die Reaktionszeiten betragen 1 bis 180 Stunden, bevorzugt 1 bis 48 Stunden, besonders bevorzugt 1 bis 8 Stunden. Die Beendigung die Reaktion kann beispielsweise mittels DC-Kontrolle bestimmt werden.

Die Ausgangsverbindungen für die Verfahrensvarianten e) sind, sofern nicht käuflich, auf einfache Weise nach literaturbekannten Verfahren herstellbar. Beispielsweise erhält man die Sulfonsäurehalogenide der Formel $Y\text{-}SO_2R^6$ durch radikalische Umsetzung von Alkanen mit Chlor und $SO_2$ oder durch Halogenierung von Aromaten mit Halogensulfonsäure $Y\text{-}SO_3H$.

Die antivirale Aktivität der erfindungsgemäßen Verbindungen I, II und III wurde in Zellkulturen, die mit Testviren infiziert wurden, getestet. Dabei zeigte sich, daß die erfindungsgemäßen Derivate eine hervorragende antivirale Wirkung zeigen.

Die erfindungsgemäßen Verbindungen sind aufgrund ihrer pharmakologischen Eigenschaften für die Behandlung von bakteriellen Erkrankungen und Viruserkrankungen, die hervorgerufen werden beispielsweise durch HSV I, II (Herpes simplex I- oder II-Virus) oder auch Picorna- und Retroviren, wie HIV (Human Immunodeficiency Virus), geeignet.

Die Erfindung betrifft daher weiter die Anwendung der erfindungsgemäßen Verbindungen der Formel I, II und III bei der Behandlung und Prophylaxe von bakteriellen Erkrankungen oder von Herpes-Virus-,

Picorna- und Retroviren-Erkrankungen.

Die Verbindungen können entweder allein oder mit physiologisch verträglichen Hilfs- oder Trägerstoffen vermischt als Arzneimittel angewandt werden. Sie können zu diesem Zweck oral in Dosen von 0,01 - 5,0 mg/kg/Tag, vorzugsweise 0,01 - 1,0 mg/kgTag oder parenteral subkutan in Dosen von 0,001 - 2,5 mg/kg/Tag, vorzugsweise 0,001 -1,0 mg/kg/Tag, insbesondere 0,005 - 0,2 mg/kg/Tag, appliziert werden. Besonders bevorzugt ist die topische Anwendung, wobei die Wirkstoffkonzentration in den Salben 0,001 - 1 %, bevorzugt 0,01 - 0,1 % beträgt. Die Dosierung kann in schweren Fällen auch erhöht werden. In vielen Fällen genügen jedoch auch geringere Dosen. Die Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der Formel I, II und/oder III enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt. Bei der topischen Anwendung reichen schon Wirkstoffkonzentrationen von 0,001 - 1 %, bevorzugt 0,01 -0,1 % aus.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfstoffen und anderen Wirkstoffträgern auch Antioxidantien, Dispergiermittel, Emulgatoren, Entschäumer, Geschmackkorrigentien, Konservireungsmitteln, Lösungsmittler oder Farbstoffe.

Die Wirkstoffe können oral, parenteral (subkutan), topisch oder rectal appliziert werden, wobei die topische Applikation bevorzugt ist.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen, Cremes oder Salben. Als inerte Trägerstoffe können z. B. Gummi arabicum, Magnesia, Magnesiumcarbonat, Kaliumphosphat, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. in Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

**Beispiele**

**Verfahren a**

12 mmol Elaiophylin werden in 120 ml Wasser, 80 ml Ethanol und 70 ml Essigester mit 25 g $KHCO_3$ bei 65 °C erhitzt. Anschließend wird im Vakuum eingeengt, die verbleibende wäßrige Phase wird mit Essigester (4x150 ml) extrahiert. Nach dem Trocknen über Natriumsulfat wird im Vakuum eingeengt. Der verbleibende Rückstand wird an Kieselgel chromatographiert (Elution mit $CHCl_3/CH_3OH$ wie 40 : 1).

**Verfahren b**

1 mmol Elaiophylin-Derivat wird in 30 ml Ethanol mit 300 ml Ethanthiol und 300 ml Triethylamin bei 60 °C 1 Stunde gerührt. Nach der Zugabe von 50 ml Wasser wird mit Essigester extrahiert (3x50 ml). Die Essigesterphase wird mit mit 30 ml Wasser gewaschen (3x) und über Natriumsulfat getrocknet. Nach dem

Eindampfen im Vakuum wird der Rückstand an Kieselgel chromatographiert (Essigester/Hexan wie 1 : 4).

**Verfahren c**

1 mmol Elaiophylin-Derivat 1 oder 5 wird in 30 ml Essigester gelöst und mit 10 Gew.-% Pd/Tierkohle versetzt. Die Hydrierung erfolgt bei Zimmertemperatur unter Rühren in einer geschlossenen Hydrierapparatur unter Einhaltung eines leichten Wasserstoffüberdrucks von bis zu 0,2 bar. Nach 3 Stunden wird abfiltriert und das Lösungsmittel entfernt. Chromatographie an Kieselgel mit Essigester/Hexan wie 1 : 3 auf 3 : 1 ergibt die entsprechenden Produkte.

**Verfahren d**

1 mmol Elaiophylin-Derivat wird in 20 ml Pyridin gelöst und mit 10 ml Säureanhydrid bei Zimmertemperatur für 24 Stunden gerührt. Nach der Hydrolyse mit 60 ml Wasser wird mit Essigester (3 x 30 ml) extrahiert. Die organische Phase wird mit 10 ml 1N HCl und 20 ml Wasser gewaschen und über Natriumsulfat getrocknet. Nach dem Eindampfen im Rotationsverdampfer bis zur Trockne im Vakuum wird an Kieselgel chromatographiert (Elution $CHCL_3/CH_3OH$ von 30 : 1 auf 10 : 1).

**Verfahren e**

1 mmol Elaiophylin-Derivat wird in 20 ml Pyridin gelöst und mit 10 mmol Methansulfonsäurechlorid bei Zimmertemperatur für eine Stunde gerührt. Nach der Hydrolyse mit 60 ml Wasser wird wie in Verfahren d aufgearbeitet.

| Physiko-chemische Daten der hergestellten Verbindungen | | | |
|---|---|---|---|
| Nr. | $MNa^+$ | M ber. | $^{13}C$ in $CDCl_3$ |
| 1 | 898 | 876,3 | 202,6, 168,4, 168,0, 148,2, 145,4, 145,3, 145,1, 144,9, 131,6, 131,4, 122,2, 122,1, 100,29, 94,2 |
| 2 | 1067 | 1045,6 | |
| 3 | 1359 | 1337,6 | |
| 4 | 1901 | 1379,5 | |
| 5 | 751 | 728.9 | 202,6, 168,0, 148,3, 145,2,.245,0, 131,6, 131,4, 122,3, 76,8, 72,2, 68,9 |
| 6 | 919 | 897,1 | 200,4, 170,3, 170,1, 167,4, 146,0, 145,8, 144,4, 132,4, 131,7, 122,0, 75,2, 74,4, |
| 7 | 959 | 937,2 | 202,23, 168,9, 167,9, 165,9, 150,0, 149, 149,3, 76,8, 72,8, 72,0 |
| 8 | 907 | 885,1 | 202,0, 168,0, 145,4, 145,1, 143,8, 132,8, 131,4, 122,2 80,9, 76,9, 71,9 |
| 9 | 891* | 852,2 | 168,5, 195,4, 195,0, 131,5, 122,0, 99,2, 76,9, 70,1, 68,3 |
| 10 | 907* | 884,2 | 168,5, 145,3, 145,0, 131,5, 122,0, 99,1, 76,9, 70,8, 68,3, 62,4, |
| 11 | 763 | 741,06 | Schmp. 152 - 153 °C |
| 12 | 908 | 886.2 | |

* $MK^+$

| Beispiele | | | | | | | | | |
|---|---|---|---|---|---|---|---|---|---|
| Nr. | Formel | $R_1$ | $R_2$ | $R_3'$ | $R_3$ | Synth. aus Reagenz | Reaktionszeit in h | Verfahren | Ausb. |
| 1 | II | H | H | - | - | ELA $KHCO_3$ | 6 | a | 44 % |
| 2 | II | Ac | Ac | - | - | 1 $Ac_2O$ | 24 | d | 85 % |
| 3 | II | Bz | H | - | - | 1 $Bz_2O$ | 24 | d | 72 % |
| 4 | II | Bz | Ac | - | - | 3 $Ac_2O$ | 24 | d | 70 % |
| 5 | III | H | H | - | - | ELA $KHCO_3$ | 14 | a | 75 % |
| 6 | III | Ac | Ac | - | - | 5 $Ac_2O$ | 24 | d | 85 % |
| 7 | III | Bz | H | - | - | 5 $Bz_2O$ | 24 | d | 78 % |
| 8 | III | $CH_3SO_2$ | H | - | - | 5 $CH_3SO_2Cl$ | 11 | e | 92 % |
| 9 | I | - | - | $SCH_2CH_3$ | $SCH_2CH_3$ | 5 $CH_3CH_2SH$ | 1 | b | 88 % |
| 10 | I | - | - | $SCH_2CH_2OH$ | $SCH_2CH_2OH$ | 5 $HOCH_2CH_2SH$ | 1 | b | 86 % |
| 11 | I hydriert | - | - | H | H | 5 $Pd/H_2$ | 3 | c | 92 % |
| 12 | I hydriert | - | - | H | L-Desoxyfucose | 5 Pd/12 | 3 | c | 94 % |
| Ac = Acetyl<br>Bz = Benzoyl | | | | | | | | | |

## Antivirale Wirksamkeit

Antivirale Aktivität in der Zellkultur

Die Prüfsubstanzen wurden in Zellkulturmedium (Dulbecco's MEM) gelöst und in einer geometrischen Verdünnungsreihe, Faktor 3, in Standard-Mikrotiterplatten in 100 $\mu$l Zellkulturmedium vorgelegt. Anschließend erfolgte die Zugabe von 100 $\mu$l einer Suspension von HeLa- bzw. Vero-Zellen in Medium mit 5 % fötalem Kälberserum in einer Zelldichte von 2 x $10^5$ Zellen/ml. Die Ansätze wurden mit 50 $\mu$l einer Suspension des jeweiligen Test-Virus infiziert, die so eingestellt war, daß die Zellen innerhalb von 72 h einen cytopathogenen Effekt zeigten. Die Auswertung erfolgte durch mikroskopische Begutachtung des Zellrasens und photometrische Messung der Neutralrotaufhame (Farbtest nach Finther). Als MHK wurde die Konzentration des Pärparats angenommen ($\mu$g/ml), bei der etwa 50 % der Zellen die Infektion überlebten (MIC = minimum inhibition concentration = MHK = minimale Hemmkonzentration).

In Tabelel 1 ist die Wirkung (Angabe der MHK in $\mu$g/ml) von verschiedenen erfindungsgemäßen Verbindungen gegen folgende Viren aufgeführt:
Herpes Simplex Virus I, Herpes Simplex Virus II

Tabelle 1

| Verbindung Nr. | Herpes I | Herpes II |
|---|---|---|
| 1 | 4,94 | 4,94 |
| 9 | 44,4 | 14,8 |
| 10 | 4,94 | 1,65 |

## Ansprüche

1. Verbindung I

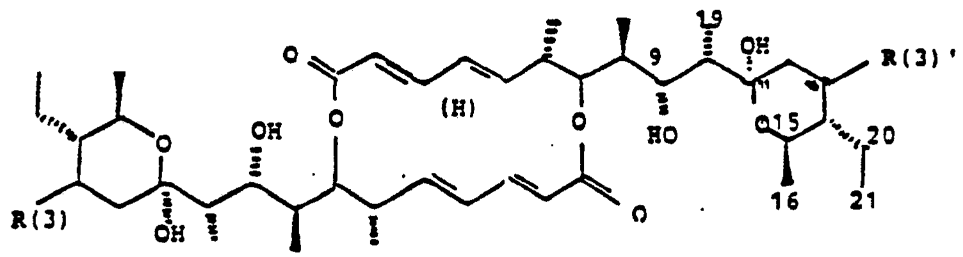

I

wobei die C-C-Doppelbindungen in dem Macrodiolidring der Verbindung der Formel I auch hydriert sein können, in welcher Formel I bedeuten:

a) R(3) und R(3′), gleich oder verschieden, einen Rest der Formel IV -SR(4) (IV)

mit R(4) gleich Wasserstoff, ($C_1$-$C_{10}$)-Alkyl, das unsubstituiert oder mit OH oder COOH substituiert ist, ($C_2$-$C_{10}$)-Alkenyl, ($C_3$-$C_8$)-Cycloalkyl, Pyrrolyl, Benzpyrrolyl, Imidazolyl, Benzimidazolyl, Triazolyl, Tetrazolyl, Phenyl, wobei die aromatischen oder Heteroarylreste unsubstituiert sind oder durch ($C_1$-$C_4$)-Alkyl, ($C_1$-$C_4$)-Alkoxy, ($C_1$-$C_4$)-Alkylcarbonyl, Carboxyl, F, Cl, Br, I, $NO_2$ oder CN ein- oder zweifach substituiert sind.

b) R(3) L-Desoxyfucose und R(3)′ wie unter a) definiert.

c) wenn die C-C-Doppelbindungen im Makrodilidring hydriert sind:

R(3) und R(3)′ Wasserstoff oder

R(3) L-Desoxyfucose und R(3)′ Wasserstoff

2. Verbindung I nach Anspruch 1, dadurch gekennzeichnet, daß R(3) und R(3)′ einen Rest der Formel IV darstellen mit R(4) gleich Wasserstoff, ($C_1$-$C_5$)-Alkyl, das unsubstituiert oder mit OH oder COOH substituiert ist,

($C_2$-$C_5$)-Alkenyl, ($C_5$-$C_8$)-Cycloalkyl, Pyrrolyl, Benzpyrrolyl, Imidazolyl, Phenyl, das unsubstituiert ist oder substituiert durch ($C_1$-$C_4$)-Alkyl, ($C_1$-$C_4$)-Alkoxy, F, Cl, Br, $NO_2$ oder CN.

3. Verbindung I nach Anspruch 1, dadurch gekennzeichnet, daß R(3) die L-Desoxyfucosylgruppe und R-(3)′ ein Rest SR(4) IV,

wie in Anspruch 1 definiert ist.

4. Verbindung I nach Anspruch 1, in der der Makrodiolidring hydriert ist, dadurch gekennzeichnet, daß R(3) der L-Desoxyfucosylrest oder Wasserstoff und R(3)′ Wasserstoff ist.

5. Verfahren zum Herstellen einer Verbindung I nach Anspruch 1, dadurch gekennzeichnet, daß man

a) Elaiophylin

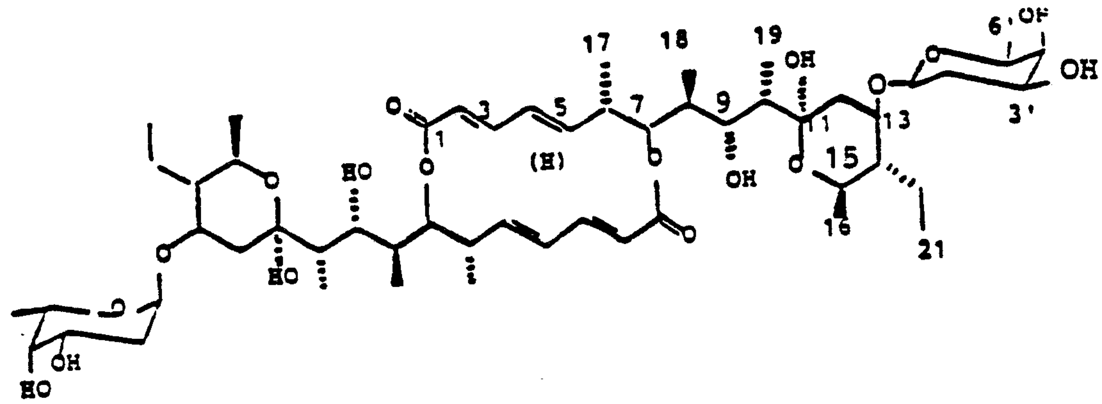

mit einer Base in wässrigem Lösungsmittel bei einem pH-Wert von 8 bis 11 zu einer Verbindung II

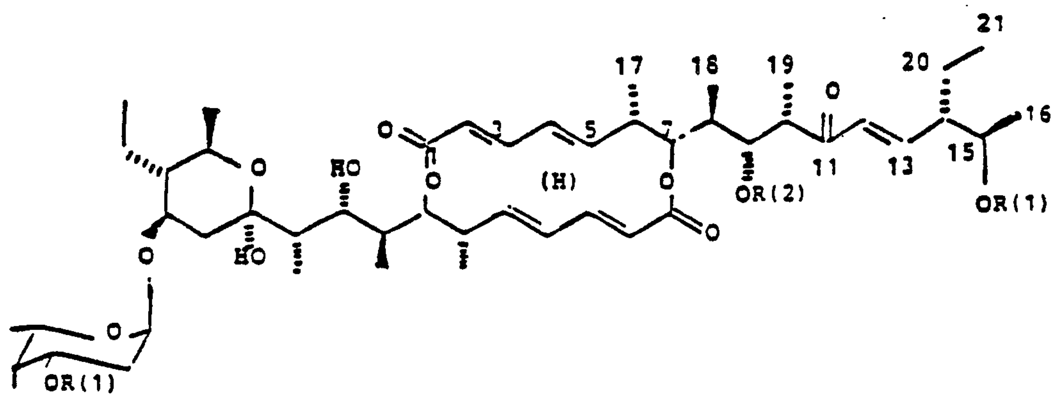

II

oder III

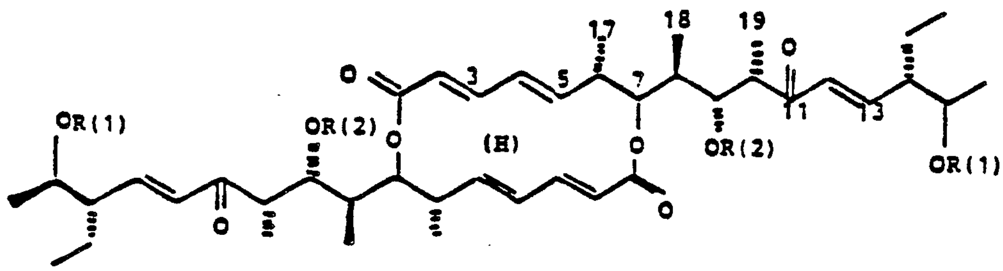

III

umsetzt

und die so erhaltene Verbindung II oder III in der R(1) = R(2) = H ist, mit einem Thiol entweder der Formel IV

HSR(4) IV,

in der R(4) wie in Anspruch 1 definiert ist, zu einer Verbindung I umsetzt

oder

die so erhaltene Verbindung II oder III mit R(1) = R(2) = H vollständig hydriert, wobei man aus Verbindungen II Verbindungen I mit R(3) = L-Desoxyfucose und R(3)' gleich Wasserstoff und aus Verbindungen III mit R(1)- = R(2) = Wasserstoff Verbindungen I mit R(3) = R(3)' = Wasserstoff erhält;

oder daß man

b) eine Verbindung II

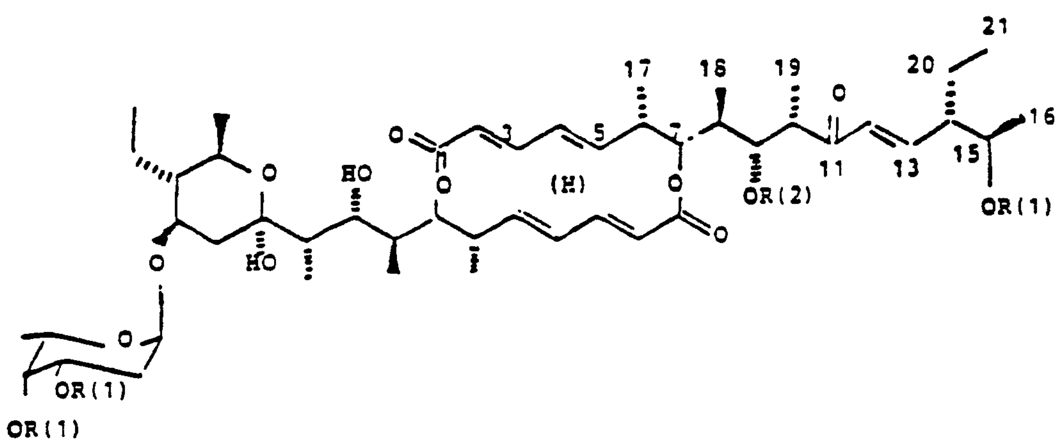

II

oder III

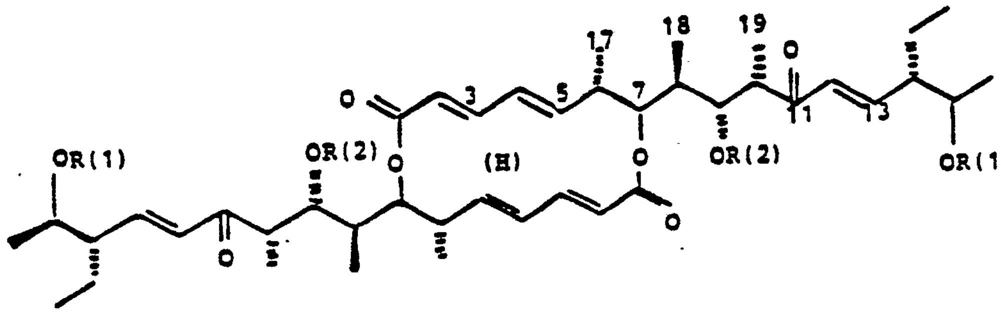

III,

in der R(1) = R(2) = H ist, mit einem Thiol der Formel
HSR(4)
umsetzt, in welchem R(4) wie in Anspruch 1 definiert ist, wobei man aus Verbindung II die Verbindung I mit R(3) = L-Desoxyfucose und R(3)′ = SR(4) IV erhält; und aus Verbindung III die Verbindung I mit R(3) = R(3)′ = SR(4); oder daß man

c) eine Verbindung II oder III, in der die C-C-Doppelbindungen in dem Makrodiolidring auch hydriert sein können, mit R(1) = R(2) = H vollständig hydriert, wobei man aus Verbindungen II Verbindungen I mit R(3) = L-Desoxyfucose und R(3)′ gleich Wasserstoff und aus Verbindungen III mit R(1) = R(2) = Wasserstoff Verbindungen I mit R(3) = R(3)′ = Wasserstoff erhält.

6. Verbindung III

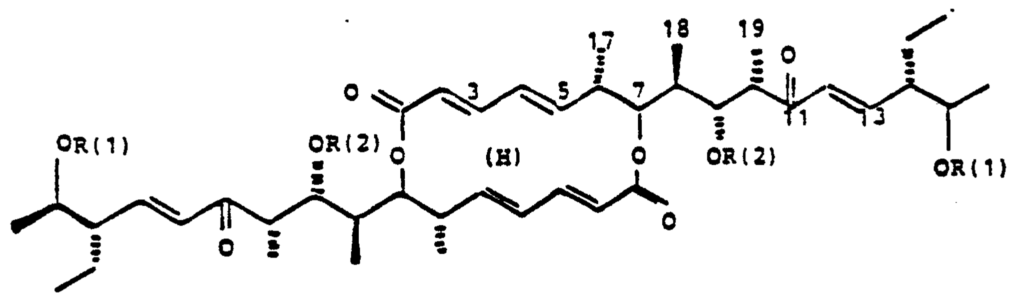

III

in welcher bedeutet:
R(1) und R(2), gleich oder verschieden, Wasserstoff, einem Rest der Formel V oder V′

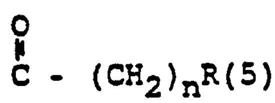

V

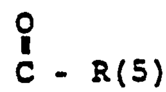

V'

mit n = 1-3 und
R(5) gleich ($C_1$-$C_5$)-Alkyl, ($C_2$-$C_{15}$)-Alkenyl, ($C_2$-$C_{15}$)-Alkinyl, ($C_3$-$C_9$)-Cycloalkyl, Aryl, Heteroaryl, unsubstituiert oder durch Halogen, $NO_2$, CN, OH, COOH, ($C_1$-$C_4$)-Alkyl oder ($C_1$-$C_4$-Alkoxy substituiert, und wobei für den Fall R(1) gleich H auch R(2) gleich H ist,
einen Rest der Formel VI
$SO_2R(6)$ VI
mit R(6) gleich ($C_1$-$C_{10}$)-Alkyl, Phenyl, Tolyl, wobei für den Fall R(1) gleich H auch R(2) gleich H ist.

7. Verbindung II

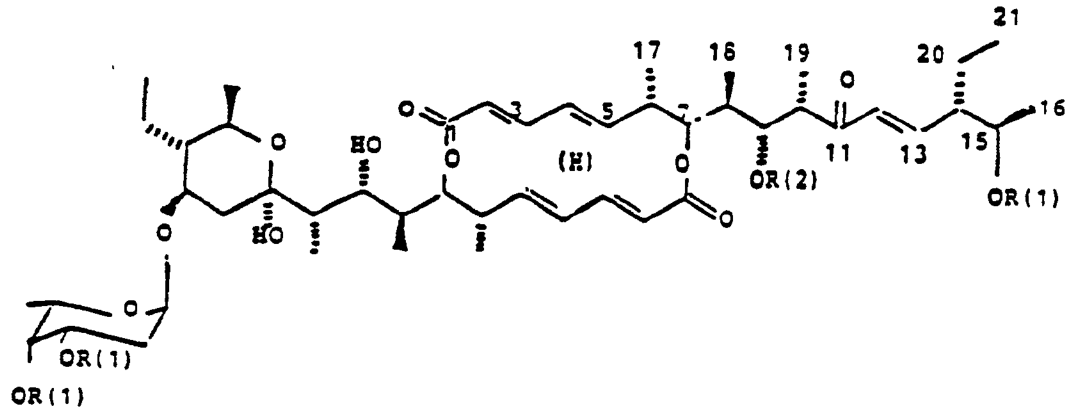

II

in welcher bedeutet:
R(1) und R(2), gleich oder verschieden, Wasserstoff, einem Rest der Formel V oder V′

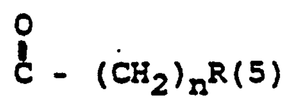

V

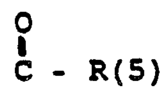

V'

mit n = 1-3 und
R(5) gleich ($C_1$-$C_5$)-Alkyl, ($C_2$-$C_{15}$)-Alkenyl, ($C_2$-$C_{15}$)-Alkinyl, ($C_3$-$C_9$)-Cycloalkyl, Aryl, Heteroaryl, unsubstituiert oder durch Halogen, $NO_2$, CN, OH, COOH, ($C_1$-$C_4$)-Alkyl oder ($C_1$-$C_4$-Alkoxy substituiert, und wobei für den Fall R(1) gleich H auch R(2) gleich H ist,
einen Rest der Formel VI
$SO_2R(6)$ VI
mit R(6) gleich ($C_1$-$C_{10}$)-Alkyl, Phenyl, Tolyl, wobei für den Fall R(1) gleich H auch R(2) gleich H ist.

8. Verbindung I nach Anspruch 1 zur Anwendung als Arzneimittel.
9. Verbindung I nach Anspruch 1 zur Anwendung als antibakteriell und antiviral wirkendes Arzneimittel.
10. Verbindung II nach Anspruch 7 zur Anwendung als Arzneimittel.
11. Verbindung II nach Anspruch 7 zur Anwendung als aktibakteriell und antiviral wirkendes Arzneimittel.
12. Verbindung III nach Anspruch 6 zur Anwendung als Arzneimittel.
13. Verbindung III nach Anspruch 6 zur Anwendung als antibakteriell und antiviral wirkendes Arzneimittel.